(19) Europäisches Patentamt
European Patent Office
Office européen des brevets

(11) Veröffentlichungsnummer: **0 331 061**
**B1**

(12) # EUROPÄISCHE PATENTSCHRIFT

(45) Veröffentlichungstag der Patentschrift: **04.07.90**

(51) Int. Cl.$^5$: **C07C 251/72**, C07C 69/738, A01N 37/10

(21) Anmeldenummer: **89103363.1**

(22) Anmeldetag: **25.02.89**

(54) **Substituierte Hydrazone und diese enthaltende Fungizide.**

(30) Priorität: **03.03.88 DE 3806874**

(43) Veröffentlichungstag der Anmeldung:
**06.09.89 Patentblatt 89/36**

(45) Bekanntmachung des Hinweises auf die Patenterteilung:
**04.07.90 Patentblatt 90/27**

(84) Benannte Vertragsstaaten:
**AT BE CH DE ES FR GB GR IT LI NL SE**

(56) Entgegenhaltungen:
**DE-A-1 768 136**
**DE-A-1 768 137**
**DE-A-2 118 468**
**US-A-4 163 062**

**CHEMICAL ABSTRACTS Band 98, Nr. 8, 25. April 1983, Seite 614, Zusammenfassung Nr. 125811v, Columbus, Ohio, USA; D. NARDI et al.: "Nucleophilic opening of the hetero-ring of N-acylisatins by acylhydrazines."**

(73) Patentinhaber: **BASF Aktiengesellschaft, Carl-Bosch-Strasse 38, D-6700 Ludwigshafen(DE)**

(72) Erfinder: **Wenderoth, Bernd, Dr., Schwalbenstrasse 26, D-6840 Lampertheim(DE)**
Erfinder: **Brand, Siegbert, Dr., Hegelstrasse 39, D-6940 Weinheim(DE)**
Erfinder: **Schuetz, Franz, Dr., Budapester Strasse 45, D-6700 Ludwigshafen(DE)**
Erfinder: **Sauter, Hubert, Dr., Neckarpromenade 20, D-6800 Mannheim 1(DE)**
Erfinder: **Ammermann, Eberhard, Dr., Sachsenstrasse 3, D-6700 Ludwigshafen(DE)**
Erfinder: **Lorenz, Gisela, Dr., Erlenweg 13, D-6730 Neustadt(DE)**

**EP 0 331 061 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Hydrazonderivate, ihre Herstellung und ihre Verwendung als Fungizide.

Es ist bekannt, N-Tridecyl-2,6-dimethylmorpholin als Fungizid zu verwenden (DE-1 164 152). Seine Wirkung ist jedoch in manchen Fällen ungenügend.

Es wurde nun gefunden, daß neue Hydrazonderivate der Formel I

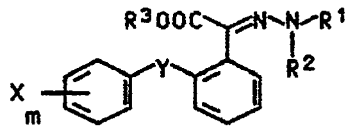

(I)

in der
$R^1$, $R^2$ und $R^3$ gleich oder verschieden sind und Wasserstoff oder Alkyl mit 1 bis 5 C-Atomen bedeuten,
X (m = 1 bis 5) gleiche oder verschiedene Substituenten Halogen, Cyano, Trifluormethyl, Nitro, $C_1$-$C_4$-Alkyl, $C_1$-$C_4$-Alkoxy, gegebenenfalls substituiertes Phenyl, gegebenenfalls substituiertes Phenoxy, gegebenenfalls substituiertes Benzyloxy oder Wasserstoff bedeutet und
Y Methylenoxy, Oxymethylen, Ethylen, Ethenylen, Ethinylen, Carboxymethylen, Carbonylamino, Methylenamino oder Sauerstoff bedeutet,
neben einer sehr hohen fungitoxischen Wirkung auch eine sehr gute Pflanzenverträglichkeit besitzen.

Die neuen Verbindungen der Formel I fallen bei der Herstellung aufgrund der C=N-Doppelbindung zum Teil als E/Z-Isomerengemische an, die in üblicher Weise, z.B. durch Kristallisation oder Chromatographie, in die einzelnen Komponenten getrennt werden können. Sowohl die einzelnen isomeren Verbindungen als auch ihre Gemische werden von der Erfindung umfaßt.

$R^1$ bedeutet bevorzugt Wasserstoff, $C_1$-$C_5$-Alkyl, Methyl, Ethyl, n-Propyl, Isopropyl, n-Butyl, sek.-Butyl, Isobutyl, tert.-Butyl, n-Pentyl und Neopentyl,
$R^2$ steht bevorzugt für Wasserstoff, $C_1$-$C_5$-Alkyl, Methyl, Ethyl, n-Propyl, Isopropyl, n-Butyl, sek.-Butyl, Isobutyl, tert.-Butyl, n-Pentyl und Neopentyl.
$R^3$ bedeutet bevorzugt Wasserstoff, $C_1$-$C_4$-Alkyl, Methyl, Ethyl, Isopropyl und n-Butyl.
m ist bevorzugt 1, 2 oder 3.
X ist bevorzugt Wasserstoff, 2-Fluor-, 3-Fluor-, 4-Fluor-, 2-Chlor-6-fluor-, 2-Chlor-, 3-Chlor-, 4-Chlor-, 2-Brom-, 3-Brom-, 4-Brom-, 2,4-Dichlor-, 2,6-Dichlor-, 3,5-Dichlor-, 2,4,6-Trichlor-, 2-Chlor-4-methyl-, 2-Methyl-4-chlor-, 2-Methyl-, 3-Methyl-, 4-Methyl-, 4-Ethyl-, 4-Isopropyl-, 4-tert.-Butyl-2,4-Dimethyl-, 2,6-Dimethyl-, 2,4,6-Trimethyl-, 2-Methoxy-4-methyl-, 4-Methoxy-2-methyl, 2-Methoxy-, 3-Methoxy-, 4-Methoxy-, 4-Ethoxy-, 4-Isopropoxy-, 2-Trifluormethyl-, 3-Trifluormethyl-, 4-Trifluormethyl-, 2-Cyano-, 4-Cyano-, 3-Nitro-, 4-Nitro-, 4-Phenyl, 4-Benzyloxy-, 4-Phenoxy, Halogenphenoxy, 4-(2-Chlor)-phenoxy-, 4-(2,4-Dichlor)-phenoxy-, $C_1$-$C_4$-Alkylphenoxy, 4-(2-Methyl)-phenoxy-, 3-Benzyloxy-, Halogen-benzyloxy-, 3-(2-Chlor)-benzyloxy-, 3-(2,4-Dichlor)-benzyloxy-, 3-(2-Fluor)-benzyloxy-, 3-(4-Brom)-benzyloxy-, $C_1$-$C_4$-Alkylbenzyloxy-, 3-(2-Methyl)-benzyloxy-, 3-Phenoxy-, 3-(2-Chlor)-phenoxy-, 3-(2,4-Dichlor)-phenoxy-, 3-(2-Fluor)-phenoxy-, 3-(4-Brom)-phenoxy, 3-(2-Methyl)-phenoxy-,
Y ist bevorzugt eine $-CH_2O-$, $-OCH_2-$, $-CH_2-CH_2-$, $-CH=CH-$, $-C\equiv C-$, $-CO_2-CH_2-$, $-CO-NH-$, $-CH_2-NH-$Gruppe oder steht für 0.

Die neuen Verbindungen lassen sich herstellen, indem man einen α-Ketocarbonsäureester der Formel II

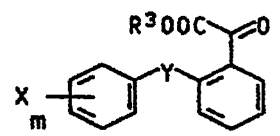

(II)

in der $X_m$, Y und $R^3$ die oben angeführten Bedeutungen haben, mit substituierten Hydrazinen der allgemeinen Formel III, in der $R^1$ und $R^2$ die oben genannten Bedeutungen haben,

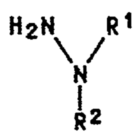

(III)

in Gegenwart einer Protonsäure (z. B. Salzsäure) in einem Lösungsmittel (z.B. Methanol) umsetzt (vgl. H. Neunhoeffer, M. Neunhoeffer, W. Litzius, Liebigs Ann. Chem. 722, 29-37 (1969)).

Die α-Ketocarbonsäureester der Formel II lassen sich z.B. aus den entsprechenden Grignard-Verbindungen, z.B.

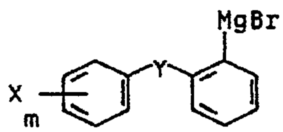

mit Imidazoliden der Formel IV herstellen (J.S. Nimitz, H.S. Mosher, J. Org. Chem. 1981, 46, 211-213),

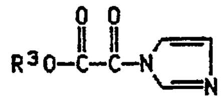

(IV)

wobei X, m, Y und $R^3$ die obengenannten Bedeutungen haben.

Ein weiteres Herstellverfahren für α-Ketocarbonsäureester der Formel II (Y = -CO-NH- oder -$CH_2$-NH-) ist z. B. das folgende:
Isatin wird mit gegebenenfalls substituierten Benzoylhalogeniden bzw. gegebenenfalls substituierten Benzylhalogeniden in Gegenwart einer Base wie zum Beispiel Natriumhydrid in einem Lösungsmittel wie zum Beispiel N,N-Dimethylformamid zu N-substituierten Isatinen der Formeln V und VI umgesetzt (vgl. G. Tacconi, P.P. Righetti, G. Desimoni, J. prakt. Chem. 315, 339-344 (1973)),

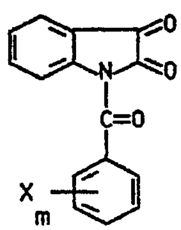

(V)

N-Benzoylisatin

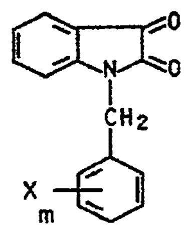

(VI)

N-Benzylisatin

wobei X und m die obengenannten Bedeutungen haben. Durch Erhitzen in $R^3$-OH, wobei $R^3$ die obengenannte Bedeutung hat, in Gegenwart einer Lewis- oder Protonsäure wie zum Beispiel Titantetrachlorid oder HCl erhält man die α-Ketocarbonsäureester der Formel II:

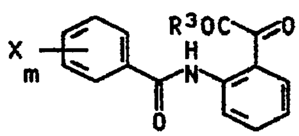

(II), Y = —CO—NH—

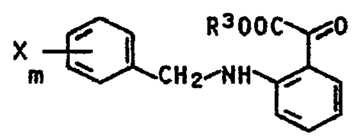

(II), Y = —$CH_2$—NH—

Ein weiteres Herstellungsverfahren für α-Ketocarbonsäureester der Formel II (Y = -$CO_2$-$CH_2$-) ist z.B. das folgende:
α-(2-Brommethylphenyl)-ß-methoxyacrylsäuremethylester (vgl. DE-3 545 318, DE-3 545 319, DE-3 620 860) wird in einem Lösungsmittel

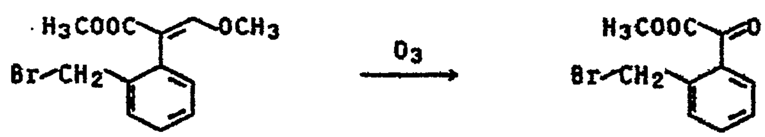


(VII)

(z.B. Methanol) einer Ozonolyse unterworfen (vgl. Bailey, Ozonation in Organic Chemistry, Academic Press, N.Y., 1982) und man erhält die neue Verbindung 2-(Brommethyl)-phenylglyoxylsäure-methylester VII. Diese Verbindung ist ein wertvolles Zwischenprodukt für die Herstellung der neuen Hydrazone.

Durch Umsetzung von Verbindung VII mit bekannten Carboxylaten der Formel VIII

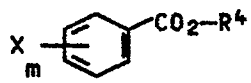

(VIII)

wobei X und m die obengenannten Bedeutungen haben und $R^4$ z.B. für Natrium oder Kalium steht, in einem Lösungsmittel wie z.B. N,N-Dimethylformamid (vgl. Synthesis 1975, 805-807), erhält man die neuen α-Ketocarbonsäureester der Formel II (Y = $-CO_2-CH_2-$).

Durch Umsetzung von Verbindung VII mit Phenol-Derivaten der Formel IX (vgl. Houben-Weyl, Methoden der organischen Chemie VI/3, 54 ff. (1965)),

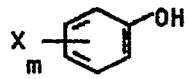

(IX)

wobei X und m die obengenannten Bedeutungen haben, in einem Lösungsmittel (z. B. Methanol), erhält man in Gegenwart einer Base wie z.B. Natriumcarbonat die α-Ketocarbonsäureester der Formel II (Y = $-OCH_2-$).

Ein weiteres Herstellungsverfahren für den 2-(Brommethyl)-phenylglyoxylsäuremethylester VII ist z.B. das folgende:
Die Bromierung des bekannten α-Ketocarbonsäureesters der Formel X (vgl. z.B. J.M. Photis, Tetrahedron Lett. 1980, 3539)

(X) 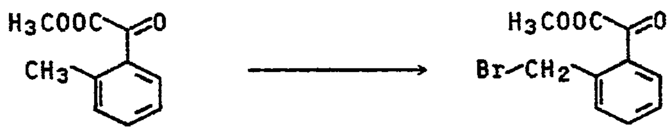 (VII)

mit Brom in einem Lösungsmittel wie z.B. Tetrachlormethan, gegebenenfalls unter Belichtung mit einer Lichtquelle (z.B. Hg-Dampf-Lampe, 300 W) oder die Bromierung mit N-Bromsuccinimid (Horner, Winkelmann, Angew. Chem. 71, 349 (1959)) führt zur Verbindung VII.

Die Herstellung der Verbindungen der Formeln I, II und VII wird durch folgende Beispiele erläutert:

1. Herstellung von 2-(Brommethyl)-phenylglyoxylsäuremethylester

1.1 2,85 g (10 mmol) α-(2-Brommethylphenyl)-ß-methoxyacrylsäuremethylester werden bei -78 °C in 30 ml Dichlormethan/Methanol (1/1) gelöst. Unter Rühren wird für eine Stunde bis zur leichten Blaufärbung Ozon eingeleitet (Ozongenerator Fischer OZ 501, 60 l $O_2$/h).

Dann werden 1,24 g (20 mmol) Dimethylsulfid zugegeben und man läßt über Nacht auf Raumtemperatur (20 °C) erwärmen. Dann wird auf ein Gemisch aus Diethylether, n-Hexan und Wasser gegossen (1:1:1). Die organische Phase wird abgetrennt, über Natriumsulfat getrocknet und eingeengt. Man erhält die obengenannte Verbindung als gelbes Öl.

1.2 5,34 g (30 mmol) 2-Methylphenylglyoxylsäuremethylester und 5,34 g (30 mmol) N-Bromsuccinimid werden in 1000 ml Tetrachlormethan für eine Stunde mit einer 300 W-Hg-Dampf-Lampe bestrahlt. Dann wird die organische Phase 1x mit Wasser und 3x mit Natriumhydrogencarbonat-Lösung gewaschen und über Natriumsulfat/Natriumcarbonat getrocknet. Nach dem Einengen wird das Rohprodukt an Kieselgel mit Methyl-t.-Butylether/n-Hexan (1/9) chromatographiert. Man erhält 3,8 g (49 %) der obengenannten Verbindung als gelbes Öl.

Die Verbindung VII zeigt folgende physikalischen Werte:
$^1$H-NMR ($CDCl_3$):
δ = 3,97 (S, 3H), 4,90 (S, 2H), 7,4-7,8 (m, 4H).
IR (Film):
2955, 1740, 1689, 1435, 1318, 1207, 999 $cm^{-1}$.

2. 2-(Benzoylamino)-phenylglyoxylsäuremethylester

2.1 Herstellung von N-Benzoylisatin

2,6 g (0,11 mol) Natriumhydrid werden in 100 ml N,N-Dimethylformamid unter Rühren mit 14,7 g (0,10 mol) Isatin (gelöst in 100 ml N,N-Dimethylformamid) versetzt. Nach einer Stunde Rühren bei Raumtemperatur werden bei 0 °C 14,1 g (0,10 mol) Benzoylchlorid zugetropft. Dann wird 10 min. bei 0 °C gerührt und auf Eis gegossen. Der ausgefallene Niederschlag wird abgesaugt. Nach dem Trocknen erhält man 20 g (80 %) N-Benzoylisatin als gelbe Kristalle.
IR (Film):
2470, 1776, 1746, 1687, 1605, 1465, 1336, 1287, 761.

2.2 Herstellung von 2-(Benzoylamino)-phenylglyoxylsäuremethylester

13 g (52 mmol) N-Benzoylisatin werden zusammen mit einem Tropfen konzentrierter Salzsäure in 100 ml Methanol für acht Stunden unter Rückfluß erhitzt. Nach dem Abkühlen wird am Rotationsverdampfer eingeengt und man erhält 14 g (95 %) des obengenannten α-Ketocarbonsäureesters als gelbe Kristalle mit folgenden physikalischen Werten:
$^1$H-NMR ($CDCl_3$):
δ = 4,03 (S, 3H), 7,22 (m, 1H), 7,55 (m, 3H), 7,76 (m, 3H) 8,10 (d, 2H), 9,05 (d, 1H), 12,10 (S, 1H).
IR (Film):
3315, 1733, 1647, 1583, 1535, 1450, 1297, 1210, 1159, 694.

3. Herstellung von 2-(Benzyloxy)-phenylglyoxylsäuremethylester

0,1 mol einer aus 1-Benzyloxy-2-brombenzol und Magnesiumspänen in Tetrahydrofuran hergestellten Grignard-Verbindung werden unter Stickstoff bei -50 °C zu 14,6 g (95 mmol) Methyloxalylimidazol in Tetrahydrofuran langsam zugetropft. Über einen Zeitraum von 4 Stunden läßt man die Mischung langsam auf Raumtemperatur (20 °C) kommen. Man gießt sie auf Eiswasser und extrahiert mehrmals mit Ether. Die vereinigten Etherphasen werden neutral gewaschen und getrocknet. Nach dem Abdampfen des Lösungsmittels wird das Produkt mit n-Pentan zur Kristallisation gebracht. Man erhält 16 g (62 %) farblose Kristalle der obengenannten Verbindung.
$^1$H-NMR ($CDCl_3$):
δ = 3,35 (s, 3H), 5,07 (s, 2H), 7,05 (m, 2H), 7,40 (m, 5H), 7,55 (m, 1H), 7,90 (m, 1H)

4. Herstellung von 2-(Benzyloxy)-phenylglyoxylsäuremethylester-N-methylhydrazon (Verbindung Nr. 1 der Tabelle)

13,5 g (50 mmol) 2-(Benzyloxy)-phenylglyoxylsäuremethylester, 2,3 g (50 mmol) Methylhydrazin und 25 ml 2n-HCl werden in 250 ml Methanol drei Tage bei Raumtemperatur gerührt. Nach dem Einengen wird in Essigester aufgenommen, mit verdünnter Natriumhydrogencarbonat-Lösung und anschließend mit Wasser gewaschen. Nach dem Trocknen über Natriumsulfat wird eingeengt. Das Rohprodukt wird über eine Kieselgelsäule (Cyclohexan/Essigester = 9/1) chromatographiert. Man erhält 2,7 g (18 %) des obengenannten Hydrazons als gelbes Öl.
$^1$H-NMR ($CDCl_3$):
δ = 3,25 (S, 3H) 3,55 (S, 3H), 5,05 (S, 2H), 6,85-7,05 (m, 2H), 7,2-7,4 (m 6H).
In entsprechender Weise lassen sich die folgenden Verbindungen herstellen.

Tabelle

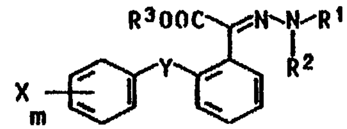

(I)

| Verb.-Nr. | $X_m$ | Y | $R^1$ | $R^2$ | $R^3$ | Fp(°C) IR($cm^{-1}$) |
|---|---|---|---|---|---|---|
| 1 | H | $-CH_2O-$ | $CH_3$ | H | $CH_3$ | 3265,1671,1489,1434, 1320,1221,1159,759 |
| 2 | 2-F | $-CH_2O-$ | $CH_3$ | H | $CH_3$ | |
| 3 | 3-F | $-CH_2O-$ | $CH_3$ | H | $CH_3$ | |
| 4 | 4-F | $-CH_2O-$ | $CH_3$ | H | $CH_3$ | |
| 5 | 2-Cl, 6-F | $-CH_2O-$ | $CH_3$ | H | $CH_3$ | |
| 6 | 2-Cl | $-CH_2O-$ | $CH_3$ | H | $CH_3$ | 3320,1681,1546,1437, 1327,1256,1162,756 |
| 7 | 3-Cl | $-CH_2O-$ | $CH_3$ | H | $CH_3$ | |
| 8 | 4-Cl | $-CH_2O-$ | $CH_3$ | H | $CH_3$ | |
| 9 | 2-Br | $-CH_2O-$ | $CH_3$ | H | $CH_3$ | |
| 10 | 3-Br | $-CH_2O-$ | $CH_3$ | H | $CH_3$ | |
| 11 | 4-Br | $-CH_2O-$ | $CH_3$ | H | $CH_3$ | |
| 12 | 2,4-$Cl_2$ | $-CH_2O-$ | $CH_3$ | H | $CH_3$ | |
| 13 | 2,6-$Cl_2$ | $-CH_2O-$ | $CH_3$ | H | $CH_3$ | |
| 14 | 3,5-$Cl_2$ | $-CH_2O-$ | $CH_3$ | H | $CH_3$ | |
| 15 | 2,4,6-$Cl_3$ | $-CH_2O-$ | $CH_3$ | H | $CH_3$ | |
| 16 | 2-Cl, 4-$CH_3$ | $-CH_2O-$ | $CH_3$ | H | $CH_3$ | |

| Verb.-Nr. | $X_m$ | Y | $R^1$ | $R^2$ | $R^3$ | Fp(°C) IR($cm^{-1}$) |
|---|---|---|---|---|---|---|
| 17 | 2-$CH_3$, 4-Cl | -$CH_2$O- | $CH_3$ | H | $CH_3$ | |
| 18 | 2-$CH_3$ | -$CH_2$O- | $CH_3$ | H | $CH_3$ | |
| 19 | 3-$CH_3$ | -$CH_2$O- | $CH_3$ | H | $CH_3$ | |
| 20 | 4-$CH_3$ | -$CH_2$O- | $CH_3$ | H | $CH_3$ | |
| 21 | 4-$C_2H_5$ | -$CH_2$O- | $CH_3$ | H | $CH_3$ | |
| 22 | 4-i-$C_3H_7$ | -$CH_2$O- | $CH_3$ | H | $CH_3$ | |
| 23 | 4-t-$C_4H_9$ | -$CH_2$O- | $CH_3$ | H | $CH_3$ | |
| 24 | 2,4-$(CH_3)_2$ | -$CH_2$O- | $CH_3$ | H | $CH_3$ | |
| 25 | 2,6-$(CH_3)_2$ | -$CH_2$O- | $CH_3$ | H | $CH_3$ | |
| 26 | 2,4,6-$(CH_3)_3$ | -$CH_2$O- | $CH_3$ | H | $CH_3$ | |
| 27 | 2-$OCH_3$, 4-$CH_3$ | -$CH_2$O- | $CH_3$ | H | $CH_3$ | |
| 28 | 4-$OCH_3$, 2-$CH_3$ | -$CH_2$O- | $CH_3$ | H | $CH_3$ | |
| 29 | 2-$OCH_3$ | -$CH_2$O- | $CH_3$ | H | $CH_3$ | |
| 30 | 3-$OCH_3$ | -$CH_2$O- | $CH_3$ | H | $CH_3$ | 113-115;3315,1675, 1545,1436,1328,1270,1230, 1160,1040,768 |
| 31 | 4-$OCH_3$ | -$CH_2$O- | $CH_3$ | H | $CH_3$ | |
| 32 | 4-$OC_2H_5$ | -$CH_2$O- | $CH_3$ | H | $CH_3$ | |
| 33 | 4-O-i-$C_3H_7$ | -$CH_2$O- | $CH_3$ | H | $CH_3$ | |
| 34 | 2-$CF_3$ | -$CH_2$O- | $CH_3$ | H | $CH_3$ | |
| 35 | 3-$CF_3$ | -$CH_2$O- | $CH_3$ | H | $CH_3$ | |
| 36 | 4-$CF_3$ | -$CH_2$O- | $CH_3$ | H | $CH_3$ | |

| Verb.-Nr. | $X_m$ | Y | $R^1$ | $R^2$ | $R^3$ | Fp(°C) IR($cm^{-1}$) |
|---|---|---|---|---|---|---|
| 37 | 2-CN | $-CH_2O-$ | $CH_3$ | H | $CH_3$ | |
| 38 | 4-CN | $-CH_2O-$ | $CH_3$ | H | $CH_3$ | |
| 39 | 3-$NO_2$ | $-CH_2O-$ | $CH_3$ | H | $CH_3$ | |
| 40 | 4-$NO_2$ | $-CH_2O-$ | $CH_3$ | H | $CH_3$ | |
| 41 | 4-$C_6H_5$ | $-CH_2O-$ | $CH_3$ | H | $CH_3$ | |
| 42 | H | $-OCH_2-$ | $CH_3$ | H | $CH_3$ | 3275,2955,1673,1598, 1528,1496,1219,1153,755 |
| 43 | 2-F | $-OCH_2-$ | $CH_3$ | H | $CH_3$ | |
| 44 | 3-F | $-OCH_2-$ | $CH_3$ | H | $CH_3$ | |
| 45 | 4-F | $-OCH_2-$ | $CH_3$ | H | $CH_3$ | |
| 46 | 2-Cl, 6-F | $-OCH_2-$ | $CH_3$ | H | $CH_3$ | |
| 47 | 2-Cl | $-OCH_2-$ | $CH_3$ | H | $CH_3$ | 3310,2950,1707,1485 1245,1158,750 |
| 48 | 3-Cl | $-OCH_2-$ | $CH_3$ | H | $CH_3$ | |
| 49 | 4-Cl | $-OCH_2-$ | $CH_3$ | H | $CH_3$ | |
| 50 | 2-Br | $-OCH_2-$ | $CH_3$ | H | $CH_3$ | |
| 51 | 3-Br | $-OCH_2-$ | $CH_3$ | H | $CH_3$ | |
| 52 | 4-Br | $-OCH_2-$ | $CH_3$ | H | $CH_3$ | |
| 53 | 2,4-$Cl_2$ | $-OCH_2-$ | $CH_3$ | H | $CH_3$ | |
| 54 | 2,6-$Cl_2$ | $-OCH_2-$ | $CH_3$ | H | $CH_3$ | |
| 55 | 3,5-$Cl_2$ | $-OCH_2-$ | $CH_3$ | H | $CH_3$ | |
| 56 | 2,4,6-$Cl_3$ | $-OCH_2-$ | $CH_3$ | H | $CH_3$ | |
| 57 | 2-Cl, 4-$CH_3$ | $-OCH_2-$ | $CH_3$ | H | $CH_3$ | |

| Verb.-Nr. | $X_m$ | Y | $R^1$ | $R^2$ | $R^3$ | Fp(°C) IR($cm^{-1}$) |
|---|---|---|---|---|---|---|
| 58 | 2-$CH_3$, 4-Cl | -$OCH_2$- | $CH_3$ | H | $CH_3$ | |
| 59 | 2-$CH_3$ | -$OCH_2$- | $CH_3$ | H | $CH_3$ | |
| 60 | 3-$CH_3$ | -$OCH_2$- | $CH_3$ | H | $CH_3$ | |
| 61 | 4-$CH_3$ | -$OCH_2$- | $CH_3$ | H | $CH_3$ | 3320,2920,1684,1508,<br>1437,1330,1233,1165,1014,760 |
| 62 | 4-$C_2H_5$ | -$OCH_2$- | $CH_3$ | H | $CH_3$ | |
| 63 | 4-i-$C_3H_7$ | -$OCH_2$- | $CH_3$ | H | $CH_3$ | |
| 64 | 4-t-$C_4H_9$ | -$OCH_2$- | $CH_3$ | H | $CH_3$ | |
| 65 | 2,4-$(CH_3)_2$ | -$OCH_2$- | $CH_3$ | H | $CH_3$ | |
| 66 | 2,6-$(CH_3)_2$ | -$OCH_2$- | $CH_3$ | H | $CH_3$ | |
| 67 | 2,4,6-$(CH_3)_3$ | -$OCH_2$- | $CH_3$ | H | $CH_3$ | |
| 68 | 2-$OCH_3$, 4-$CH_3$ | -$OCH_2$- | $CH_3$ | H | $CH_3$ | |
| 69 | 4-$OCH_3$, 2-$CH_3$ | -$OCH_2$- | $CH_3$ | H | $CH_3$ | |
| 70 | 2-$OCH_3$ | -$OCH_2$- | $CH_3$ | H | $CH_3$ | |
| 71 | 3-$OCH_3$ | -$OCH_2$- | $CH_3$ | H | $CH_3$ | |
| 72 | 4-$OCH_3$ | -$OCH_2$- | $CH_3$ | H | $CH_3$ | |
| 73 | 4-$OC_2H_5$ | -$OCH_2$- | $CH_3$ | H | $CH_3$ | |
| 74 | 4-O-i$C_3H_7$ | -$OCH_2$- | $CH_3$ | H | $CH_3$ | |
| 75 | 2-$CF_3$ | -$OCH_2$- | $CH_3$ | H | $CH_3$ | |
| 76 | 3-$CF_3$ | -$OCH_2$- | $CH_3$ | H | $CH_3$ | |
| 77 | 4-$CF_3$ | -$OCH_2$- | $CH_3$ | H | $CH_3$ | |
| 78 | 2-CN | -$OCH_2$- | $CH_3$ | H | $CH_3$ | |

| Verb.-Nr. | $X_m$ | Y | $R^1$ | $R^2$ | $R^3$ | Fp(°C) | IR($cm^{-1}$) |
|---|---|---|---|---|---|---|---|
| 79 | 4-CN | $-OCH_2-$ | $CH_3$ | H | $CH_3$ | | |
| 80 | $3-NO_2$ | $-OCH_2-$ | $CH_3$ | H | $CH_3$ | | |
| 81 | $4-NO_2$ | $-OCH_2-$ | $CH_3$ | H | $CH_3$ | | |
| 82 | $4-C_6H_5$ | $-OCH_2-$ | $CH_3$ | H | $CH_3$ | | |
| 83 | H | -CH=CH- | $CH_3$ | H | $CH_3$ | | |
| 84 | 2-F | -CH=CH- | $CH_3$ | H | $CH_3$ | | |
| 85 | 3-F | -CH=CH- | $CH_3$ | H | $CH_3$ | | |
| 86 | 4-F | -CH=CH- | $CH_3$ | H | $CH_3$ | | |
| 87 | 2-Cl, 6-F | -CH=CH- | $CH_3$ | H | $CH_3$ | | |
| 88 | 2-Cl | -CH=CH- | $CH_3$ | H | $CH_3$ | | |
| 89 | 3-Cl | -CH=CH- | $CH_3$ | H | $CH_3$ | | |
| 90 | 4-Cl | -CH=CH- | $CH_3$ | H | $CH_3$ | | |
| 91 | 2-Br | -CH=CH- | $CH_3$ | H | $CH_3$ | | |
| 92 | 3-Br | -CH=CH- | $CH_3$ | H | $CH_3$ | | |
| 93 | 4-Br | -CH=CH- | $CH_3$ | H | $CH_3$ | | |
| 94 | $2,4-Cl_2$ | -CH=CH- | $CH_3$ | H | $CH_3$ | | |
| 95 | $2,6-Cl_2$ | -CH=CH- | $CH_3$ | H | $CH_3$ | | |
| 96 | $3,5-Cl_2$ | -CH=CH- | $CH_3$ | H | $CH_3$ | | |
| 97 | $2,4,6-Cl_3$ | -CH=CH- | $CH_3$ | H | $CH_3$ | | |
| 98 | $2-CH_3$, 4-Cl | -CH=CH- | $CH_3$ | H | $CH_3$ | | |
| 99 | 2-Cl, $4-CH_3$ | -CH=CH- | $CH_3$ | H | $CH_3$ | | |
| 100 | $2-CH_3$ | -CH=CH- | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 101 | 3-$CH_3$ | -CH=CH- | $CH_3$ | H | $CH_3$ | | |
| 102 | 4-$CH_3$ | -CH=CH- | $CH_3$ | H | $CH_3$ | | |
| 103 | 4-$C_2H_5$ | -CH=CH- | $CH_3$ | H | $CH_3$ | | |
| 104 | 4-i-$C_3H_7$ | -CH=CH- | $CH_3$ | H | $CH_3$ | | |
| 105 | 4-t-$C_4H_9$ | -CH=CH- | $CH_3$ | H | $CH_3$ | | |
| 106 | 2,4-$(CH_3)_2$ | -CH=CH- | $CH_3$ | H | $CH_3$ | | |
| 107 | 2,6-$(CH_3)_2$ | -CH=CH- | $CH_3$ | H | $CH_3$ | | |
| 108 | 2,4,6-$(CH_3)_3$ | -CH=CH- | $CH_3$ | H | $CH_3$ | | |
| 109 | 2-$OCH_3$, 4-$CH_3$ | -CH=CH- | $CH_3$ | H | $CH_3$ | | |
| 110 | 4-$OCH_3$, 2-$CH_3$ | -CH=CH- | $CH_3$ | H | $CH_3$ | | |
| 111 | 2-$OCH_3$ | -CH=CH- | $CH_3$ | H | $CH_3$ | | |
| 112 | 3-$OCH_3$ | -CH=CH- | $CH_3$ | H | $CH_3$ | | |
| 113 | 4-$OCH_3$ | -CH=CH- | $CH_3$ | H | $CH_3$ | | |
| 114 | 4-$OC_2H_5$ | -CH=CH- | $CH_3$ | H | $CH_3$ | | |
| 115 | 4-O-i-$C_3H_7$ | -CH=CH- | $CH_3$ | H | $CH_3$ | | |
| 116 | 2-$CF_3$ | -CH=CH- | $CH_3$ | H | $CH_3$ | | |
| 117 | 3-$CF_3$ | -CH=CH- | $CH_3$ | H | $CH_3$ | | |
| 118 | 4-$CF_3$ | -CH=CH- | $CH_3$ | H | $CH_3$ | | |
| 119 | 2-CN | -CH=CH- | $CH_3$ | H | $CH_3$ | | |
| 120 | 4-CN | -CH=CH- | $CH_3$ | H | $CH_3$ | | |
| 121 | 3-$NO_2$ | -CH=CH- | $CH_3$ | H | $CH_3$ | | |
| 122 | 4-$NO_2$ | -CH=CH- | $CH_3$ | H | $CH_3$ | | |
| 123 | 4-$C_6H_5$ | -CH=CH- | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 124 | H | $-CH_2-CH_2$ | $CH_3$ | H | $CH_3$ | | |
| 125 | 2-F | $-CH_2-CH_2-$ | $CH_3$ | H | $CH_3$ | | |
| 126 | 3-F | $-CH_2-CH_2-$ | $CH_3$ | H | $CH_3$ | | |
| 127 | 4-F | $-CH_2-CH_2-$ | $CH_3$ | H | $CH_3$ | | |
| 128 | 2-Cl, 6-F | $-CH_2-CH_2-$ | $CH_3$ | H | $CH_3$ | | |
| 129 | 2-Cl | $-CH_2-CH_2-$ | $CH_3$ | H | $CH_3$ | | |
| 130 | 3-Cl | $-CH_2-CH_2-$ | $CH_3$ | H | $CH_3$ | | |
| 131 | 4-Cl | $-CH_2-CH_2-$ | $CH_3$ | H | $CH_3$ | | |
| 132 | 2-Br | $-CH_2-CH_2-$ | $CH_3$ | H | $CH_3$ | | |
| 133 | 3-Br | $-CH_2-CH_2-$ | $CH_3$ | H | $CH_3$ | | |
| 134 | 4-Br | $-CH_2-CH_2-$ | $CH_3$ | H | $CH_3$ | | |
| 135 | 2,4-$Cl_2$ | $-CH_2-CH_2-$ | $CH_3$ | H | $CH_3$ | | |
| 136 | 2,6-$Cl_2$ | $-CH_2-CH_2-$ | $CH_3$ | H | $CH_3$ | | |
| 137 | 3,5-$Cl_2$ | $-CH_2-CH_2-$ | $CH_3$ | H | $CH_3$ | | |
| 138 | 2,4,6-$Cl_3$ | $-CH_2-CH_2-$ | $CH_3$ | H | $CH_3$ | | |
| 139 | 2-Cl, 4-$CH_3$ | $-CH_2-CH_2-$ | $CH_3$ | H | $CH_3$ | | |
| 140 | 2-$CH_3$, 4-Cl | $-CH_2-CH_2-$ | $CH_3$ | H | $CH_3$ | | |
| 141 | 2-$CH_3$ | $-CH_2-CH_2-$ | $CH_3$ | H | $CH_3$ | | |
| 142 | 3-$CH_3$ | $-CH_2-CH_2-$ | $CH_3$ | H | $CH_3$ | | |
| 143 | 4-$CH_3$ | $-CH_2-CH_2-$ | $CH_3$ | H | $CH_3$ | | |
| 144 | 4-$C_2H_5$ | $-CH_2-CH_2-$ | $CH_3$ | H | $CH_3$ | | |
| 145 | 4-i-$C_3H_7$ | $-CH_2-CH_2-$ | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 146 | 4-t-$C_4H_9$ | $-CH_2-CH_2-$ | $CH_3$ | H | $CH_3$ | | |
| 147 | 2,4-$(CH_3)_2$ | $-CH_2-CH_2-$ | $CH_3$ | H | $CH_3$ | | |
| 148 | 2,6-$(CH_3)_2$ | $-CH_2-CH_2-$ | $CH_3$ | H | $CH_3$ | | |
| 149 | 2,4,6-$(CH_3)_3$ | $-CH_2-CH_2-$ | $CH_3$ | H | $CH_3$ | | |
| 150 | 2-$OCH_3$, 4-$CH_3$ | $-CH_2-CH_2-$ | $CH_3$ | H | $CH_3$ | | |
| 151 | 4-$OCH_3$, 2-$CH_3$ | $-CH_2-CH_2-$ | $CH_3$ | H | $CH_3$ | | |
| 152 | 2-$OCH_3$ | $-CH_2-CH_2-$ | $CH_3$ | H | $CH_3$ | | |
| 153 | 3-$OCH_3$ | $-CH_2-CH_2-$ | $CH_3$ | H | $CH_3$ | | |
| 154 | 4-$OCH_3$ | $-CH_2-CH_2-$ | $CH_3$ | H | $CH_3$ | | |
| 155 | 4-$OC_2H_5$ | $-CH_2-CH_2-$ | $CH_3$ | H | $CH_3$ | | |
| 156 | 4-O-i-$C_3H_7$ | $-CH_2-CH_2-$ | $CH_3$ | H | $CH_3$ | | |
| 157 | 2-$CF_3$ | $-CH_2-CH_2-$ | $CH_3$ | H | $CH_3$ | | |
| 158 | 3-$CF_3$ | $-CH_2-CH_2-$ | $CH_3$ | H | $CH_3$ | | |
| 159 | 4-$CF_3$ | $-CH_2-CH_2-$ | $CH_3$ | H | $CH_3$ | | |
| 160 | 2-CN | $-CH_2-CH_2-$ | $CH_3$ | H | $CH_3$ | | |
| 161 | 4-CN | $-CH_2-CH_2-$ | $CH_3$ | H | $CH_3$ | | |
| 162 | 3-$NO_2$ | $-CH_2-CH_2-$ | $CH_3$ | H | $CH_3$ | | |
| 163 | 4-$NO_2$ | $-CH_2-CH_2-$ | $CH_3$ | H | $CH_3$ | | |
| 164 | 4-$C_6H_5$ | $-CH_2-CH_2-$ | $CH_3$ | H | $CH_3$ | | |
| 165 | H | Ethinylen | $CH_3$ | H | $CH_3$ | | |
| 166 | 2-F | Ethinylen | $CH_3$ | H | $CH_3$ | | |
| 167 | 2-Cl | Ethinylen | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 168 | 2-Br | Ethinylen | $CH_3$ | H | $CH_3$ | | |
| 169 | 4-Br | Ethinylen | $CH_3$ | H | $CH_3$ | | |
| 170 | 2-$CH_3$ | Ethinylen | $CH_3$ | H | $CH_3$ | | |
| 171 | 4-$CH_3$ | Ethinylen | $CH_3$ | H | $CH_3$ | | |
| 172 | 2-$OCH_3$ | Ethinylen | $CH_3$ | H | $CH_3$ | | |
| 173 | 4-$CF_3$ | Ethinylen | $CH_3$ | H | $CH_3$ | | |
| 174 | 2-$NO_2$ | Ethinylen | $CH_3$ | H | $CH_3$ | | |
| 175 | H | O | $CH_3$ | H | $CH_3$ | | |
| 176 | 2-F | O | $CH_3$ | H | $CH_3$ | | |
| 177 | 2-Cl | O | $CH_3$ | H | $CH_3$ | | |
| 178 | 2-Br | O | $CH_3$ | H | $CH_3$ | | |
| 179 | 4-Br | O | $CH_3$ | H | $CH_3$ | | |
| 180 | 4-Cl | O | $CH_3$ | H | $CH_3$ | | |
| 181 | 2-$CH_3$ | O | $CH_3$ | H | $CH_3$ | | |
| 182 | 4-$CH_3$ | O | $CH_3$ | H | $CH_3$ | | |
| 183 | 2-$OCH_3$ | O | $CH_3$ | H | $CH_3$ | | |
| 184 | 4-$OCH_3$ | O | $CH_3$ | H | $CH_3$ | | |
| 185 | 4-$C_6H_5$ | O | $CH_3$ | H | $CH_3$ | | |
| 186 | H | $-CH_2O-$ | H | H | H | | |
| 187 | H | $-OCH_2-$ | H | H | H | | |
| 188 | 4-$OCH_2$-$C_6H_5$ | $-CH_2O-$ | $CH_3$ | H | $CH_3$ | | |
| 189 | 4-$OCH_2$-$C_6H_5$ | $-OCH_2-$ | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 190 | 4-$OC_6H_5$ | $-CH_2O-$ | $CH_3$ | H | $CH_3$ | | |
| 191 | 4-$OC_6H_5$ | $-OCH_2-$ | $CH_3$ | H | $CH_3$ | | |
| 192 | 4–O– 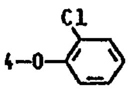 | $-CH_2O-$ | $CH_3$ | H | $CH_3$ | | |
| 193 | 4–O– 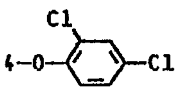 | $-CH_2O-$ | $CH_3$ | H | $CH_3$ | | |
| 194 | 4–O– 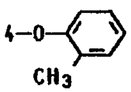 | $-CH_2O-$ | $CH_3$ | H | $CH_3$ | | |
| 195 | 3-$OCH_2$-$C_6H_5$ | $-CH_2O-$ | $CH_3$ | H | $CH_3$ | | |
| 196 | 3–$OCH_2$– 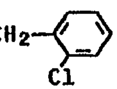 | $-CH_2O-$ | $CH_3$ | H | $CH_3$ | | |
| 197 | 3–$OCH_2$– 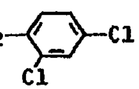 | $-CH_2O-$ | $CH_3$ | H | $CH_3$ | | |
| 198 | 3–$OCH_2$– 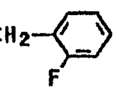 | $-CH_2O-$ | $CH_3$ | H | $CH_3$ | | |
| 199 | 3–$OCH_2$– 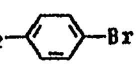 | $-CH_2O-$ | $CH_3$ | H | $CH_3$ | | |
| 200 | 3–$OCH_2$– 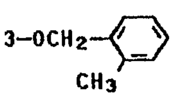 | $-CH_2O-$ | $CH_3$ | H | $CH_3$ | | |
| 201 | 3-$OC_6H_5$ | $-CH_2O-$ | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 202 | 3-O- 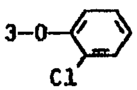 | $-CH_2O-$ | $CH_3$ | H | $CH_3$ | | |
| 203 | 3-O- 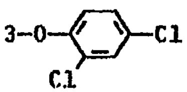 | $-CH_2O$ | $CH_3$ | H | $CH_3$ | | |
| 204 | 3-O- 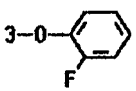 | $-CH_2O-$ | $CH_3$ | H | $CH_3$ | | |
| 205 | 3-O- 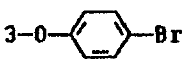 | $-CH_2O-$ | $CH_3$ | H | $CH_3$ | | |
| 206 | 3-O- 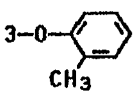 | $-CH_2O-$ | $CH_3$ | H | $CH_3$ | | |
| 207 | H | $-CH_2O-$ | $CH_3$ | H | H | | |
| 208 | H | $-CH_2O-$ | $CH_3$ | H | $C_2H_5$ | | |
| 209 | H | $-CH_2O-$ | $CH_3$ | H | $C_3H_7$ | | |
| 210 | H | $-CH_2O-$ | $CH_3$ | H | $i-C_3H_7$ | | |
| 211 | H | $-CH_2O-$ | $CH_3$ | H | $n-C_4H_9$ | | |
| 212 | H | $-OCH_2-$ | $CH_3$ | H | H | | |
| 213 | H | $-OCH_2-$ | $CH_3$ | H | $C_2H_5$ | | |
| 214 | H | $-OCH_2-$ | $CH_3$ | H | $i-C_3H_7$ | | |
| 215 | H | $-OCH_2-$ | $CH_3$ | H | $n-C_4H_9$ | | |
| 216 | H | $-CH_2O-$ | $CH_3$ | $CH_3$ | $CH_3$ | | |
| 217 | 2-F | $-CH_2O-$ | $CH_3$ | $CH_3$ | $CH_3$ | | |
| 218 | 3-F | $-CH_2O-$ | $CH_3$ | $CH_3$ | $CH_3$ | | |
| 219 | 4-F | $-CH_2O-$ | $CH_3$ | $CH_3$ | $CH_3$ | | |

| Verb.-Nr. | $X_m$ | Y | $R^1$ | $R^2$ | $R^3$ | Fp(°C) IR($cm^{-1}$) |
|---|---|---|---|---|---|---|
| 220 | 2-Cl, 6-F | $-CH_2O-$ | $CH_3$ | $CH_3$ | $CH_3$ | |
| 221 | 2-Cl | $-CH_2O-$ | $CH_3$ | $CH_3$ | $CH_3$ | 2945,1693,1556,1488,1437, 1303,1202,1072,1030,752 |
| 222 | 3-Cl | $-CH_2O-$ | $CH_3$ | $CH_3$ | $CH_3$ | |
| 223 | 4-Cl | $-CH_2O-$ | $CH_3$ | $CH_3$ | $CH_3$ | |
| 224 | 2-Br | $-CH_2O-$ | $CH_3$ | $CH_3$ | $CH_3$ | |
| 225 | 3-Br | $-CH_2O-$ | $CH_3$ | $CH_3$ | $CH_3$ | |
| 226 | 4-Br | $-CH_2O-$ | $CH_3$ | $CH_3$ | $CH_3$ | |
| 227 | 2,4-$Cl_2$ | $-CH_2O-$ | $CH_3$ | $CH_3$ | $CH_3$ | |
| 228 | 2,6-$Cl_2$ | $-CH_2O-$ | $CH_3$ | $CH_3$ | $CH_3$ | |
| 229 | 3,5-$Cl_2$ | $-CH_2O-$ | $CH_3$ | $CH_3$ | $CH_3$ | |
| 230 | 2,4,6-$Cl_3$ | $-CH_2O-$ | $CH_3$ | $CH_3$ | $CH_3$ | |
| 231 | 2-Cl, 4-$CH_3$ | $-CH_2O-$ | $CH_3$ | $CH_3$ | $CH_3$ | |
| 232 | 2-$CH_3$, 4-Cl | $-CH_2O-$ | $CH_3$ | $CH_3$ | $CH_3$ | |
| 233 | 2-$CH_3$ | $-CH_2O-$ | $CH_3$ | $CH_3$ | $CH_3$ | |
| 234 | 3-$CH_3$ | $-CH_2O-$ | $CH_3$ | $CH_3$ | $CH_3$ | |
| 235 | 4-$CH_3$ | $-CH_2O-$ | $CH_3$ | $CH_3$ | $CH_3$ | |
| 236 | 4-$C_2H_5$ | $-CH_2O-$ | $CH_3$ | $CH_3$ | $CH_3$ | |
| 237 | 4-i-$C_3H_7$ | $-CH_2O-$ | $CH_3$ | $CH_3$ | $CH_3$ | |
| 238 | 4-t-$C_4H_9$ | $-CH_2O-$ | $CH_3$ | $CH_3$ | $CH_3$ | |
| 239 | 2,4-$(CH_3)_2$ | $-CH_2O-$ | $CH_3$ | $CH_3$ | $CH_3$ | |
| 240 | 2,6-$(CH_3)_2$ | $-CH_2O-$ | $CH_3$ | $CH_3$ | $CH_3$ | |
| 241 | 2,4,6-$(CH_3)_3$ | $-CH_2O-$ | $CH_3$ | $CH_3$ | $CH_3$ | |
| 242 | 2-$OCH_3$, 4-$CH_3$ | $-CH_2O-$ | $CH_3$ | $CH_3$ | $CH_3$ | |
| 243 | 4-$OCH_3$, 2-$CH_3$ | $-CH_2O-$ | $CH_3$ | $CH_3$ | $CH_3$ | |
| 244 | 2-$OCH_3$ | $-CH_2O-$ | $CH_3$ | $CH_3$ | $CH_3$ | |

| Verb.-Nr. | $X_m$ | Y | $R^1$ | $R^2$ | $R^3$ | Fp(°C) | IR($cm^{-1}$) |
|---|---|---|---|---|---|---|---|
| 245 | 3-$OCH_3$ | $-CH_2O-$ | $CH_3$ | $CH_3$ | $CH_3$ | | |
| 246 | 4-$OCH_3$ | $-CH_2O-$ | $CH_3$ | $CH_3$ | $CH_3$ | | |
| 247 | 4-$OC_2H_5$ | $-CH_2O-$ | $CH_3$ | $CH_3$ | $CH_3$ | | |
| 248 | 4-O-i-$C_3H_7$ | $-CH_2O-$ | $CH_3$ | $CH_3$ | $CH_3$ | | |
| 249 | 2-$CF_3$ | $-CH_2O-$ | $CH_3$ | $CH_3$ | $CH_3$ | | |
| 250 | 3-$CF_3$ | $-CH_2O-$ | $CH_3$ | $CH_3$ | $CH_3$ | | |
| 251 | 4-$CF_3$ | $-CH_2O-$ | $CH_3$ | $CH_3$ | $CH_3$ | | |
| 252 | 2-CN | $-CH_2O-$ | $CH_3$ | $CH_3$ | $CH_3$ | | |
| 253 | 4-CN | $-CH_2O-$ | $CH_3$ | $CH_3$ | $CH_3$ | | |
| 254 | 3-$NO_2$ | $-CH_2O-$ | $CH_3$ | $CH_3$ | $CH_3$ | | |
| 255 | 4-$NO_2$ | $-CH_2O-$ | $CH_3$ | $CH_3$ | $CH_3$ | | |
| 256 | 4-$C_6H_5$ | $-CH_2O-$ | $CH_3$ | $CH_3$ | $CH_3$ | | |
| 257 | H | $-OCH_2-$ | $CH_3$ | $CH_3$ | $CH_3$ | | |
| 258 | 2-F | $-OCH_2-$ | $CH_3$ | $CH_3$ | $CH_3$ | | |
| 259 | 3-F | $-OCH_2-$ | $CH_3$ | $CH_3$ | $CH_3$ | | |
| 260 | 4-F | $-OCH_2-$ | $CH_3$ | $CH_3$ | $CH_3$ | | |
| 261 | 2-Cl, 6-F | $-OCH_2-$ | $CH_3$ | $CH_3$ | $CH_3$ | | |
| 262 | 2-Cl | $-OCH_2-$ | $CH_3$ | $CH_3$ | $CH_3$ | | |
| 263 | 3-Cl | $-OCH_2-$ | $CH_3$ | $CH_3$ | $CH_3$ | | |
| 264 | 4-Cl | $-OCH_2-$ | $CH_3$ | $CH_3$ | $CH_3$ | | 2930,1682,1554,1490,1231,<br>1209,1073,1026,824 |
| 265 | 2-Br | $-OCH_2-$ | $CH_3$ | $CH_3$ | $CH_3$ | | |
| 266 | 3-Br | $-OCH_2-$ | $CH_3$ | $CH_3$ | $CH_3$ | | |
| 267 | 4-Br | $-OCH_2-$ | $CH_3$ | $CH_3$ | $CH_3$ | | |
| 268 | 2,4-$Cl_2$ | $-OCH_2-$ | $CH_3$ | $CH_3$ | $CH_3$ | | |
| 269 | 2,6-$Cl_2$ | $-OCH_2-$ | $CH_3$ | $CH_3$ | $CH_3$ | | |
| 270 | 3,5-$Cl_2$ | $-OCH_2-$ | $CH_3$ | $CH_3$ | $CH_3$ | | |
| 271 | 2,4,6-$Cl_3$ | $-OCH_2-$ | $CH_3$ | $CH_3$ | $CH_3$ | | |

| Verb.-Nr. | $X_m$ | Y | $R^1$ | $R^2$ | $R^3$ | Fp(°C) IR($cm^{-1}$) |
|---|---|---|---|---|---|---|
| 272 | 2-Cl, 4-$CH_3$ | $-OCH_2-$ | $CH_3$ | $CH_3$ | $CH_3$ | |
| 273 | 2-$CH_3$, 4-Cl | $-OCH_2-$ | $CH_3$ | $CH_3$ | $CH_3$ | |
| 274 | 2-$CH_3$ | $-OCH_2-$ | $CH_3$ | $CH_3$ | $CH_3$ | |
| 275 | 3-$CH_3$ | $-OCH_2-$ | $CH_3$ | $CH_3$ | $CH_3$ | |
| 276 | 4-$CH_3$ | $-OCH_2-$ | $CH_3$ | $CH_3$ | $CH_3$ | 3420, 2950, 1681, 1555, 1507, 1438, 1303, 1223, 1026, 813 |
| 277 | 4-$C_2H_5$ | $-OCH_2-$ | $CH_3$ | $CH_3$ | $CH_3$ | |
| 278 | 4-i-$C_3H_7$ | $-OCH_2-$ | $CH_3$ | $CH_3$ | $CH_3$ | |
| 279 | 4-t-$C_4H_9$ | $-OCH_2-$ | $CH_3$ | $CH_3$ | $CH_3$ | |
| 280 | 2,4-$(CH_3)_2$ | $-OCH_2-$ | $CH_3$ | $CH_3$ | $CH_3$ | |
| 281 | 2,6-$(CH_3)_2$ | $-OCH_2-$ | $CH_3$ | $CH_3$ | $CH_3$ | |
| 282 | 2,4,6-$(CH_3)_3$ | $-OCH_2-$ | $CH_3$ | $CH_3$ | $CH_3$ | |
| 283 | 2-$OCH_3$, 4-$CH_3$ | $-OCH_2-$ | $CH_3$ | $CH_3$ | $CH_3$ | |
| 284 | 4-$OCH_3$, 2-$CH_3$ | $-OCH_2-$ | $CH_3$ | $CH_3$ | $CH_3$ | |
| 285 | 2-$OCH_3$ | $-OCH_2-$ | $CH_3$ | $CH_3$ | $CH_3$ | |
| 286 | 3-$OCH_3$ | $-OCH_2-$ | $CH_3$ | $CH_3$ | $CH_3$ | |
| 287 | 4-$OCH_3$ | $-OCH_2-$ | $CH_3$ | $CH_3$ | $CH_3$ | |
| 288 | 4-$OC_2H_5$ | $-OCH_2-$ | $CH_3$ | $CH_3$ | $CH_3$ | |
| 289 | 4-O-i$C_3H_7$ | $-OCH_2-$ | $CH_3$ | $CH_3$ | $CH_3$ | |
| 290 | 2-$CF_3$ | $-OCH_2-$ | $CH_3$ | $CH_3$ | $CH_3$ | |
| 291 | 3-$CF_3$ | $-OCH_2-$ | $CH_3$ | $CH_3$ | $CH_3$ | |
| 292 | 4-$CF_3$ | $-OCH_2-$ | $CH_3$ | $CH_3$ | $CH_3$ | |
| 293 | 2-CN | $-OCH_2-$ | $CH_3$ | $CH_3$ | $CH_3$ | |
| 294 | 4-CN | $-OCH_2-$ | $CH_3$ | $CH_3$ | $CH_3$ | |
| 295 | 3-$NO_2$ | $-OCH_2-$ | $CH_3$ | $CH_3$ | $CH_3$ | |
| 296 | 4-$NO_2$ | $-OCH_2-$ | $CH_3$ | $CH_3$ | $CH_3$ | |
| 297 | 4-$C_6H_5$ | $-OCH_2-$ | $CH_3$ | $CH_3$ | $CH_3$ | |
| 298 | H | $-CO_2-CH_2-$ | $CH_3$ | H | $CH_3$ | |

| Verb.-Nr. | $X_m$ | Y | $R^1$ | $R^2$ | $R^3$ | Fp($^oC$) | IR($cm^{-1}$) |
|---|---|---|---|---|---|---|---|
| 299 | 2-F | $-CO_2-CH_2-$ | $CH_3$ | H | $CH_3$ | | |
| 300 | 3-F | $-CO_2-CH_2-$ | $CH_3$ | H | $CH_3$ | | |
| 301 | 4-F | $-CO_2-CH_2-$ | $CH_3$ | H | $CH_3$ | | |
| 302 | 2-Cl, 6-F | $-CO_2-CH_2-$ | $CH_3$ | H | $CH_3$ | | |
| 303 | 2-Cl | $-CO_2-CH_2-$ | $CH_3$ | H | $CH_3$ | | |
| 304 | 3-Cl | $-CO_2-CH_2-$ | $CH_3$ | H | $CH_3$ | | |
| 305 | 4-Cl | $-CO_2-CH_2-$ | $CH_3$ | H | $CH_3$ | | |
| 306 | 2-Br | $-CO_2-CH_2-$ | $CH_3$ | H | $CH_3$ | | |
| 307 | 3-Br | $-CO_2-CH_2-$ | $CH_3$ | H | $CH_3$ | | |
| 308 | 4-Br | $-CO_2-CH_2-$ | $CH_3$ | H | $CH_3$ | | |
| 309 | 2,4-$Cl_2$ | $-CO_2-CH_2-$ | $CH_3$ | H | $CH_3$ | | |
| 310 | 2,6-$Cl_2$ | $-CO_2-CH_2-$ | $CH_3$ | H | $CH_3$ | | |
| 311 | 3,5-$Cl_2$ | $-CO_2-CH_2-$ | $CH_3$ | H | $CH_3$ | | |
| 312 | 2,4,6-$Cl_3$ | $-CO_2-CH_2-$ | $CH_3$ | H | $CH_3$ | | |
| 313 | 2-Cl, 4-$CH_3$ | $-CO_2-CH_2-$ | $CH_3$ | H | $CH_3$ | | |
| 314 | 2-$CH_3$, 4-Cl | $-CO_2-CH_2-$ | $CH_3$ | H | $CH_3$ | | |
| 315 | 2-$CH_3$ | $-CO_2-CH_2-$ | $CH_3$ | H | $CH_3$ | | |
| 316 | 3-$CH_3$ | $-CO_2-CH_2-$ | $CH_3$ | H | $CH_3$ | | |
| 317 | 4-$CH_3$ | $-CO_2-CH_2-$ | $CH_3$ | H | $CH_3$ | | |
| 318 | 4-$C_2H_5$ | $-CO_2-CH_2-$ | $CH_3$ | H | $CH_3$ | | |
| 319 | 4-i-$C_3H_7$ | $-CO_2-CH_2-$ | $CH_3$ | H | $CH_3$ | | |
| 320 | 4-t-$C_4H_9$ | $-CO_2-CH_2-$ | $CH_3$ | H | $CH_3$ | | |
| 321 | 2,4-$(CH_3)_2$ | $-CO_2-CH_2-$ | $CH_3$ | H | $CH_3$ | | |
| 322 | 2,6-$(CH_3)_2$ | $-CO_2-CH_2-$ | $CH_3$ | H | $CH_3$ | | |
| 323 | 2,4,6-$(CH_3)_3$ | $-CO_2-CH_2-$ | $CH_3$ | H | $CH_3$ | | |
| 324 | 2-$OCH_3$, 4-$CH_3$ | $-CO_2-CH_2-$ | $CH_3$ | H | $CH_3$ | | |
| 325 | 4-$OCH_3$, 2-$CH_3$ | $-CO_2-CH_2-$ | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 326 | 2-$OCH_3$ | $-CO_2-CH_2-$ | $CH_3$ | H | $CH_3$ | | |
| 327 | 3-$OCH_3$ | $-CO_2-CH_2-$ | $CH_3$ | H | $CH_3$ | | |
| 328 | 4-$OCH_3$ | $-CO_2-CH_2-$ | $CH_3$ | H | $CH_3$ | | |
| 329 | 4-$OC_2H_5$ | $-CO_2-CH_2-$ | $CH_3$ | H | $CH_3$ | | |
| 330 | 4-O-i-$C_3H_7$ | $-CO_2-CH_2-$ | $CH_3$ | H | $CH_3$ | | |
| 331 | 2-$CF_3$ | $-CO_2-CH_2-$ | $CH_3$ | H | $CH_3$ | | |
| 332 | 3-$CF_3$ | $-CO_2-CH_2-$ | $CH_3$ | H | $CH_3$ | | |
| 333 | 4-$CF_3$ | $-CO_2-CH_2-$ | $CH_3$ | H | $CH_3$ | | |
| 334 | 2-CN | $-CO_2-CH_2-$ | $CH_3$ | H | $CH_3$ | | |
| 335 | 4-CN | $-CO_2-CH_2-$ | $CH_3$ | H | $CH_3$ | | |
| 336 | 3-$NO_2$ | $-CO_2-CH_2-$ | $CH_3$ | H | $CH_3$ | | |
| 337 | 4-$NO_2$ | $-CO_2-CH_2-$ | $CH_3$ | H | $CH_3$ | | |
| 338 | 4-$C_6H_5$ | $-CO_2-CH_2-$ | $CH_3$ | H | $CH_3$ | | |
| 339 | H | -CH=CH- | $CH_3$ | $CH_3$ | $CH_3$ | | |
| 340 | H | $-CH_2-CH_2-$ | $CH_3$ | $CH_3$ | $CH_3$ | | |
| 341 | H | Ethinylen | $CH_3$ | $CH_3$ | $CH_3$ | | |
| 342 | H | O | $CH_3$ | $CH_3$ | $CH_3$ | | |
| 343 | H | $-CO_2-CH_2-$ | $CH_3$ | $CH_3$ | $CH_3$ | | |
| 344 | H | $-CH_2O-$ | $C_2H_5$ | H | $CH_3$ | | |
| 345 | H | $-CH_2O-$ | $C_3H_7$ | H | $CH_3$ | | |
| 346 | H | $-CH_2O-$ | i-$C_3H_7$ | H | $CH_3$ | | |
| 347 | H | $-CH_2O-$ | n-$C_4H_9$ | H | $CH_3$ | | |
| 348 | H | $-CH_2O-$ | i-$C_4H_9$ | H | $CH_3$ | | |
| 349 | H | $-CH_2O-$ | t-$C_4H_9$ | H | $CH_3$ | | |
| 350 | H | $-CH_2O-$ | $C_5H_{11}$ | H | $CH_3$ | | |
| 351 | H | $-OCH_2-$ | $C_2H_5$ | H | $CH_3$ | | |
| 352 | H | $-OCH_2-$ | $C_3H_7$ | H | $CH_3$ | | |

| Verb.-Nr. | $X_m$ | Y | $R^1$ | $R^2$ | $R^3$ | Fp(°C) | IR(cm$^{-1}$) |
|---|---|---|---|---|---|---|---|
| 353 | H | $-OCH_2-$ | $i-C_3H_7$ | H | $CH_3$ | | |
| 354 | H | $-OCH_2-$ | $n-C_4H_9$ | H | $CH_3$ | | |
| 355 | H | $-OCH_2-$ | $i-C_4H_9$ | H | $CH_3$ | | |
| 356 | H | $-OCH_2-$ | $t-C_4H_9$ | H | $CH_3$ | | |
| 357 | H | $-OCH_2-$ | $C_5H_{11}$ | H | $CH_3$ | | |
| 358 | H | $-CH_2O-$ | $C_2H_5$ | $C_2H_5$ | $CH_3$ | | |
| 359 | H | $-CO_2-CH_2-$ | $C_2H_5$ | H | $CH_3$ | | |
| 360 | H | $-CO_2-CH_2-$ | $C_3H_7$ | H | $CH_3$ | | |
| 361 | H | -CO-NH- | $CH_3$ | $CH_3$ | $CH_3$ | | |
| 362 | H | $-CH_2-NH-$ | $CH_3$ | $CH_3$ | $CH_3$ | | |
| 363 | H | -CO-NH- | $C_2H_5$ | H | $CH_3$ | | |
| 364 | H | $-CH_2-NH-$ | $C_2H_5$ | H | $CH_3$ | | |
| 365 | H | -CO-NH- | $CH_3$ | H | $CH_3$ | | |
| 366 | 2-F | -CO-NH- | $CH_3$ | H | $CH_3$ | | |
| 367 | 3-F | -CO-NH- | $CH_3$ | H | $CH_3$ | | |
| 368 | 4-F | -CO-NH- | $CH_3$ | H | $CH_3$ | | |
| 369 | 2-Cl, 6-F | -CO-NH- | $CH_3$ | H | $CH_3$ | | |
| 370 | 2-Cl | -CO-NH- | $CH_3$ | H | $CH_3$ | | |
| 371 | 3-Cl | -CO-NH- | $CH_3$ | H | $CH_3$ | | |
| 372 | 4-Cl | -CO-NH- | $CH_3$ | H | $CH_3$ | | |
| 373 | 2-Br | -CO-NH- | $CH_3$ | H | $CH_3$ | | |
| 374 | 3-Br | -CO-NH- | $CH_3$ | H | $CH_3$ | | |
| 375 | 4-Br | -CO-NH- | $CH_3$ | H | $CH_3$ | | |
| 376 | $2,4-Cl_2$ | -CO-NH- | $CH_3$ | H | $CH_3$ | | |
| 377 | $2,6-Cl_2$ | -CO-NH- | $CH_3$ | H | $CH_3$ | | |
| 378 | $3,5-Cl_2$ | -CO-NH- | $CH_3$ | H | $CH_3$ | | |
| 379 | $2,4,6-Cl_3$ | -CO-NH- | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 380 | 2-Cl, 4-$CH_3$ | -CO-NH- | $CH_3$ | H | $CH_3$ | | |
| 381 | 2-$CH_3$, 4-Cl | -CO-NH- | $CH_3$ | H | $CH_3$ | | |
| 382 | 2-$CH_3$ | -CO-NH- | $CH_3$ | H | $CH_3$ | | |
| 383 | 3-$CH_3$ | -CO-NH- | $CH_3$ | H | $CH_3$ | | |
| 384 | 4-$CH_3$ | -CO-NH- | $CH_3$ | H | $CH_3$ | | |
| 385 | 4-$C_2H_5$ | -CO-NH- | $CH_3$ | H | $CH_3$ | | |
| 386 | 4-i-$C_3H_7$ | -CO-NH- | $CH_3$ | H | $CH_3$ | | |
| 387 | 4-t-$C_4H_9$ | -CO-NH- | $CH_3$ | H | $CH_3$ | | |
| 388 | 2,4-$(CH_3)_2$ | -CO-NH- | $CH_3$ | H | $CH_3$ | | |
| 389 | 2,6-$(CH_3)_2$ | -CO-NH- | $CH_3$ | H | $CH_3$ | | |
| 390 | 2,4,6-$(CH_3)_3$ | -CO-NH- | $CH_3$ | H | $CH_3$ | | |
| 391 | 2-$OCH_3$, 4-$CH_3$ | -CO-NH- | $CH_3$ | H | $CH_3$ | | |
| 392 | 4-$OCH_3$, 2-$CH_3$ | -CO-NH- | $CH_3$ | H | $CH_3$ | | |
| 393 | 2-$OCH_3$ | -CO-NH- | $CH_3$ | H | $CH_3$ | | |
| 394 | 3-$OCH_3$ | -CO-NH- | $CH_3$ | H | $CH_3$ | | |
| 395 | 4-$OCH_3$ | -CO-NH- | $CH_3$ | H | $CH_3$ | | |
| 396 | 4-$OC_2H_5$ | -CO-NH- | $CH_3$ | H | $CH_3$ | | |
| 397 | 4-O-i-$C_3H_7$ | -CO-NH- | $CH_3$ | H | $CH_3$ | | |
| 398 | 2-$CF_3$ | -CO-NH- | $CH_3$ | H | $CH_3$ | | |
| 399 | 3-$CF_3$ | -CO-NH- | $CH_3$ | H | $CH_3$ | | |
| 400 | 4-$CF_3$ | -CO-NH- | $CH_3$ | H | $CH_3$ | | |
| 401 | 2-CN | -CO-NH- | $CH_3$ | H | $CH_3$ | | |
| 402 | 4-CN | -CO-NH- | $CH_3$ | H | $CH_3$ | | |
| 403 | 3-$NO_2$ | -CO-NH- | $CH_3$ | H | $CH_3$ | | |
| 404 | 4-$NO_2$ | -CO-NH- | $CH_3$ | H | $CH_3$ | | |
| 405 | 4-$C_6H_5$ | -CO-NH- | $CH_3$ | H | $CH_3$ | | |
| 406 | H | -$CH_2$-NH- | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 407 | 2-F | $-CH_2-NH-$ | $CH_3$ | H | $CH_3$ | | |
| 408 | 3-F | $-CH_2-NH-$ | $CH_3$ | H | $CH_3$ | | |
| 409 | 4-F | $-CH_2-NH-$ | $CH_3$ | H | $CH_3$ | | |
| 410 | 2-Cl, 6-F | $-CH_2-NH-$ | $CH_3$ | H | $CH_3$ | | |
| 411 | 2-Cl | $-CH_2-NH-$ | $CH_3$ | H | $CH_3$ | | |
| 412 | 3-Cl | $-CH_2-NH-$ | $CH_3$ | H | $CH_3$ | | |
| 413 | 4-Cl | $-CH_2-NH-$ | $CH_3$ | H | $CH_3$ | | |
| 414 | 2-Br | $-CH_2-NH-$ | $CH_3$ | H | $CH_3$ | | |
| 415 | 3-Br | $-CH_2-NH-$ | $CH_3$ | H | $CH_3$ | | |
| 416 | 4-Br | $-CH_2-NH-$ | $CH_3$ | H | $CH_3$ | | |
| 417 | 2,4-$Cl_2$ | $-CH_2-NH-$ | $CH_3$ | H | $CH_3$ | | |
| 418 | 2,6-$Cl_2$ | $-CH_2-NH-$ | $CH_3$ | H | $CH_3$ | | |
| 419 | 3,5-$Cl_2$ | $-CH_2-NH-$ | $CH_3$ | H | $CH_3$ | | |
| 420 | 2,4,6-$Cl_3$ | $-CH_2-NH-$ | $CH_3$ | H | $CH_3$ | | |
| 421 | 2-Cl, 4-$CH_3$ | $-CH_2-NH-$ | $CH_3$ | H | $CH_3$ | | |
| 422 | 2-$CH_3$, 4-Cl | $-CH_2-NH-$ | $CH_3$ | H | $CH_3$ | | |
| 423 | 2-$CH_3$ | $-CH_2-NH-$ | $CH_3$ | H | $CH_3$ | | |
| 424 | 3-$CH_3$ | $-CH_2-NH-$ | $CH_3$ | H | $CH_3$ | | |
| 425 | 4-$CH_3$ | $-CH_2-NH-$ | $CH_3$ | H | $CH_3$ | | |
| 426 | 4-$C_2H_5$ | $-CH_2-NH-$ | $CH_3$ | H | $CH_3$ | | |
| 427 | 4-i-$C_3H_7$ | $-CH_2-NH-$ | $CH_3$ | H | $CH_3$ | | |
| 428 | 4-t-$C_4H_9$ | $-CH_2-NH-$ | $CH_3$ | H | $CH_3$ | | |
| 429 | 2,4-$(CH_3)_2$ | $-CH_2-NH-$ | $CH_3$ | H | $CH_3$ | | |
| 430 | 2,6-$(CH_3)_2$ | $-CH_2-NH-$ | $CH_3$ | H | $CH_3$ | | |
| 431 | 2,4,6-$(CH_3)_3$ | $-CH_2-NH-$ | $CH_3$ | H | $CH_3$ | | |
| 432 | 2-$OCH_3$, 4-$CH_3$ | $-CH_2-NH-$ | $CH_3$ | H | $CH_3$ | | |
| 433 | 4-$OCH_3$, 2-$CH_3$ | $-CH_2-NH-$ | $CH_3$ | H | $CH_3$ | | |

| Verb.-Nr. | $X_m$ | Y | $R^1$ | $R^2$ | $R^3$ | Fp(°C) | IR($cm^{-1}$) |
|---|---|---|---|---|---|---|---|
| 434 | 2-$OCH_3$ | -$CH_2$-NH- | $CH_3$ | H | $CH_3$ | | |
| 435 | 3-$OCH_3$ | -$CH_2$-NH- | $CH_3$ | H | $CH_3$ | | |
| 436 | 4-$OCH_3$ | -$CH_2$-NH- | $CH_3$ | H | $CH_3$ | | |
| 437 | 4-$OC_2H_5$ | -$CH_2$-NH- | $CH_3$ | H | $CH_3$ | | |
| 438 | 4-O-i-$C_3H_7$ | -$CH_2$-NH- | $CH_3$ | H | $CH_3$ | | |
| 439 | 2-$CF_3$ | -$CH_2$-NH- | $CH_3$ | H | $CH_3$ | | |
| 440 | 3-$CF_3$ | -$CH_2$-NH- | $CH_3$ | H | $CH_3$ | | |
| 441 | 4-$CF_3$ | -$CH_2$-NH- | $CH_3$ | H | $CH_3$ | | |
| 442 | 2-CN | -$CH_2$-NH- | $CH_3$ | H | $CH_3$ | | |
| 443 | 4-CN | -$CH_2$-NH- | $CH_3$ | H | $CH_3$ | | |
| 444 | 3-$NO_2$ | -$CH_2$-NH- | $CH_3$ | H | $CH_3$ | | |
| 445 | 4-$NO_2$ | -$CH_2$-NH- | $CH_3$ | H | $CH_3$ | | |
| 446 | 4-$C_6H_5$ | -$CH_2$-NH- | $CH_3$ | H | $CH_3$ | | |
| 447 | 2-Cl | -$CH_2$O- | H | H | $CH_3$ | | 3418,3295,1718,1565,1491, 1435,1340,1226,1029,749 |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 30 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 42 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 30 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 42 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 30 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 42 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 30 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 42 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 30 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid, Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoff wurde N-Tridecyl-2,6-dimethylmorpholin (A) - bekannt aus DE 1 164 152 - benutzt.

Anwendungsbeispiel 1

Wirksamkeit gegen Plasmopara viticola

Blätter von Topfreben der Sorte "Müller Thurgau" wurden mit wäßriger Spritzbrühe, die 80 % (Gew.%) Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48

Stunden in einer wasserdampfgesättigten Kammer bei 24 °C und anschließend für 5 Tage in einem Gewächshaus mit Temperaturen zwischen 20 und 30 °C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 30 und 42 bei der Anwendung als 0,05 %ige Spritzbrühe eine bessere fungizide Wirkung (90 %) zeigen als der bekannte Vergleichswirkstoff A (50 %).

Anwendungsbeispiel 2

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inoculiert. Anschließend wurden die Versuchspflanzen in Klimakammern bei 22 bis 24 °C und 95 bis 99 % relativer Luftfeuchtigkeit aufgestellt. Nach 6 Tagen wurde das Ausmaß des Krankheitsbefalls ermittelt.

Das Ergebnis zeigt, daß der Wirkstoff 42 bei der Anwendung als 0,05 %ige Spritzbrühe eine bessere fungizide Wirkung (90 %) zeigt als der bekannte Vergleichswirkstoff A (50 %).

## Patentansprüche

1. Substituierte Hydrazone der Formel I

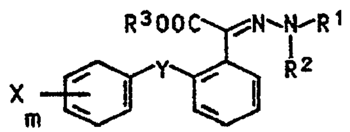

(I)

in der
$R^1$, $R^2$ und $R^3$ gleich oder verschieden sind und Wasserstoff oder Alkyl mit 1 bis 5 C-Atomen bedeuten,
X (m = 1 bis 5) gleiche oder verschiedene Substituenten Halogen, Cyano, Trifluormethyl, Nitro, $C_1$-$C_4$-Alkyl, $C_1$-$C_4$-Alkoxy, gegebenenfalls substituiertes Phenyl, gegebenenfalls substituiertes Phenoxy, gegebenenfalls substituiertes Benzyloxy oder Wasserstoff bedeutet und
Y Methylenoxy, Oxymethylen, Ethylen, Ethenylen, Ethinylen, Carboxymethylen, Carbonylamino, Methylenamino oder Sauerstoff bedeutet.

2. Verbindungen der Formel I gemäß Anspruch 1, in der X Wasserstoff, 2-Fluor-, 3-Fluor-, 4-Fluor-, 2-Chlor-6-fluor-, 2-Chlor-, 3-Chlor-, 4-Chlor-, 2-Brom-, 3-Brom-, 4-Brom-, 2,4-Dichlor-, 2,6-Dichlor-, 3,5-Dichlor-, 2,4,6-Trichlor-, 2-Chlor-4-methyl-, 2-Methyl-4-chlor-, 2-Methyl-, 3-Methyl-, 4-Methyl-, 4-Ethyl-, 4-Isopropyl-, 4-tert.-Butyl-, 2,4-Dimethyl-, 2,6-Dimethyl-, 2,4,6-Trimethyl-, 2-Methoxy-4-methyl-, 4-Methoxy-2-methyl, 2-Methoxy-, 3-Methoxy-, 4-Methoxy-, 4-Ethoxy-, 4-Isopropoxy-, 2-Trifluormethyl-, 3-Trifluormethyl-, 4-Trifluormethyl-, 2-Cyano-, 4-Cyano-, 3-Nitro-, 4-Nitro-, 4-Phenyl, 4-Benzyloxy-, 4-Phenoxy, Halogenphenoxy, 4-(2-Chlor)-phenoxy-, 4-(2,4-Dichlor)-phenoxy-, $C_1$-$C_4$-Alkylphenoxy, 4-(2-Methyl)-phenoxy-, 3-Benzyloxy-, Halogen-benzyloxy-, 3-(2-Chlor)-benzyloxy-, 3-(2,4-Dichlor)-benzyloxy-, 3-(2-Fluor)-benzyloxy-, 3-(4-Brom)-benzyloxy-, $C_1$-$C_4$-Alkylbenzyloxy-, 3-(2-Methyl)-benzyloxy-, 3-Phenoxy-, 3-(2-Chlor)-phenoxy-, 3-(2,4-Dichlor)-phenoxy-, 3-(2-Fluor)-phenoxy-, 3-(4-Brom)-phenoxy, 3-(2-Methyl)-phenoxy-,
$R^1$ Wasserstoff, Methyl, Ethyl, n-Propyl, Isopropyl, n-Butyl, sek.-Butyl, Isobutyl, tert.-Butyl, n-Pentyl oder Neopentyl,
$R^2$ Wasserstoff, Methyl, Ethyl, n-Propyl, Isopropyl, n-Butyl, sek.-Butyl, Isobutyl, tert.-Butyl, n-Pentyl oder Neopentyl,
$R^3$ Wasserstoff, Methyl, Ethyl, Isopropyl, n-Butyl und
Y eine $-CH_2O-$, $-OCH_2-$, $-CH_2-CH_2-$, $-CH=CH-$, $-C\equiv C-$, $-CO_2-CH_2-$, $-CO-NH-$, $-CH_2-NH-$Gruppe oder 0 bedeutet.

3. Fungizides Mittel, enthaltend ein substituiertes Hydrazon der Formel I

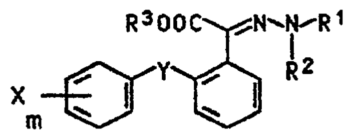

(I)

in der
$R^1$, $R^2$ und $R^3$ gleich oder verschieden sind und Wasserstoff oder Alkyl mit 1 bis 5 C-Atomen bedeuten,
X (m = 1 bis 5) gleiche oder verschiedene Substituenten Halogen, Cyano, Trifluormethyl, Nitro, $C_1$-$C_4$-

Alkyl, $C_1$-$C_4$-Alkoxy, gegebenenfalls substituiertes Phenyl, gegebenenfalls substituiertes Phenoxy, gegebenenfalls substituiertes Benzyloxy oder Wasserstoff bedeutet und
Y Methylenoxy, Oxymethylen, Ethylen, Ethenylen, Ethinylen, Carboxymethylen, Carbonylamino, Methylenamino oder Sauerstoff bedeutet
und einen flüssigen oder festen Trägerstoff.

4. Verfahren zur Bekämpfung von Pilzen, dadurch gekennzeichnet, daß man ein substituiertes Hydrazon der Formel I

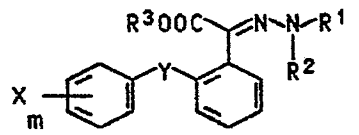

(I)

in der
$R^1$, $R^2$ und $R^3$ gleich oder verschieden sind und Wasserstoff oder Alkyl mit 1 bis 5 C-Atomen bedeuten,
X (m = 1 bis 5) gleiche oder verschiedene Substituenten Halogen, Cyano, Trifluormethyl, Nitro, $C_1$-$C_4$-Alkyl, $C_1$-$C_4$-Alkoxy, gegebenenfalls substituiertes Phenyl, gegebenenfalls substituiertes Phenoxy, gegebenenfalls substituiertes Benzyloxy oder Wasserstoff bedeutet und
Y Methylenoxy, Oxymethylen, Ethylen, Ethenylen, Ethinylen, Carboxymethylen, Carbonylamino, Methylenamino oder Sauerstoff bedeutet
auf die Pilze oder auf durch Pilzbefall bedrohte Flächen, Pflanzen, Materialien oder Saatgüter einwirken läßt.

5. 2-(Brommethyl)-phenylglyoxylsäuremethylester.

6. Verbindung gemäß Anspruch 1, dadurch gekennzeichnet, daß $R^1$ Methyl, $R^2$ Wasserstoff, $R^3$ Methyl, $X_m$ Wasserstoff und Y Methylenoxy bedeuten.

7. Verbindung gemäß Anspruch 1, dadurch gekennzeichnet, daß $R^1$ Methyl, $R^2$ Wasserstoff, $R^3$ Methyl, $X_m$ Wasserstoff und Y Oxymethylen bedeuten.

8. Verbindung gemäß Anspruch 1, dadurch gekennzeichnet, daß $R^1$ Methyl, $R^2$ Wasserstoff, $R^3$ Methyl, $X_m$ 3-Methoxy und Y Methylenoxy bedeuten.

9. Verbindung gemäß Anspruch 1, dadurch gekennzeichnet, daß $R^1$ Methyl, $R^2$ Methyl, $R^3$ Methyl, $X_m$ 4-Methyl und Y Oxymethylen bedeuten.

## Claims

1. Substituted hydrazones of the formula I

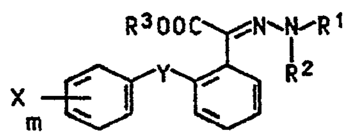

(I)

where
$R^1$, $R^2$ and $R^3$ are identical or different and each denotes hydrogen or alkyl of 1 to 5 carbon atoms,
$X_m$ (m = from 1 to 5) is from one to five identical or different substituents selected from the group consisting of halogen, cyano, trifluoromethyl, nitro, $C_1$–$C_4$-alkyl, $C_1$–$C_4$-alkoxy, substituted or unsubstituted phenyl, substituted or unsubstituted phenoxy, substituted or unsubstituted benzyloxy and hydrogen, and
Y is methyleneoxy, oxymethylene, ethylene, ethenylene, ethynylene, carboxymethylene, carbonylamino, methyleneamino or oxygen.

2. A compound of the formula I as claimed in claim 1, where X is hydrogen, 2-fluoro, 3-fluoro, 4-fluoro, 2-chloro-6-fluoro, 2-chloro, 3-chloro, 4-chloro, 2-bromo, 3-bromo, 4-bromo, 2,4-dichloro, 2,6-dichloro, 3,5-dichloro, 2,4,6-trichloro, 2-chloro-4-methyl, 2-methyl-4-chloro, 2-methyl, 3-methyl, 4-methyl, 4-ethyl, 4-isopropyl, 4-tert-butyl, 2,4-dimethyl, 2,6-dimethyl, 2,4,6-trimethyl, 2-methoxy-4-methyl, 4-methoxy-2-methyl, 2-methoxy, 3-methoxy, 4-methoxy, 4-ehtoxy, 4-isopropoxy, 2-trifluoromethyl, 3-trifluoromethyl, 4-trifluoromethyl, 2-cyano, 4-cyano, 3-nitro, 4-nitro, 4-phenyl, 4-benzyloxy, 4-phenoxy, halophenoxy, 4-(2-chloro)-phenoxy, 4-(2,4-dichloro)-phenoxy, $C_1$–$C_4$-alkylphenoxy, 4-(2-methyl)-phenoxy, 3-benzyloxy, halobenzyloxy, 3-(2-chloro)-benzyloxy, 3-(2,4-dichloro)-benzyloxy, 3-(2-fluoro)-benzyloxy, 3-(4-bromo)-benzyloxy, $C_1$–$C_4$-alkyl-benzyloxy, 3-(2-methyl)-benzyloxy, 3-phenoxy, 3-(2-chloro)-phenoxy, 3-(2,4-dichloro)-phenoxy, (3-(2-fluoro)-phenoxy, 3-(4-bromo)-phenoxy, or 3-(2-methyl)-phenoxy,
$R^1$ is hydrogen, methyl, ethyl, n-propyl, isopropl, n-butyl, sec-butyl, isobutyl, tert-butyl, n-pentyl or neopentyl,

$R^2$ is hydrogen, methyl, ethyl, n-propyl, isopropyl, n-butyl, sec-butyl, isobutyl, tert-butyl, n-pentyl or neopentyl,
$R^3$ is hydrogen, methyl, ethyl, isopropyl, n-butyl and
Y is $-CH_2O-$, $-OCH_2-$, $-CH_2-CH_2-$, $-CH=CH-$, $-C=C-$, $-CO_2-CH_2-$, $-CO-NH-$, $-CH_2-NH-$ or O.

3. A fungicidal agent containing a substituted hydrazone of the formula I

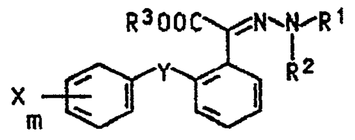

(I)

where
$R^1$, $R^2$ and $R^3$ are identical or different and each denotes hydrogen or alkyl of 1 to 5 carbon atoms,
$X_m$(m=from 1 to 5) is from one to five identical or different substituents selected from the group consisting of halogen, cyano, trifuloromethyl, nitro, $C_1$–$C_4$-alkyl, $C_1$–$C_4$-alkoxy, substituted or unsubstituted phenyl, substituted or unsubstituted phenoxy, substituted or unsubstituted benzyloxy and hydrogen, and
Y is methyleneoxy, oxymethylene, ethylene, ethenylene, ethynylene, carboxymethylene, carbonylamino, methyleneamino or oxygen, and a solid or liquid carrier.

4. A process for controlling fungi, wherein a substituted hydrazone of the formula I

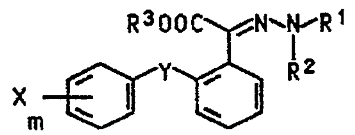

(I)

where
$R^1$, $R^2$ and $R^3$ are identical or different and each denotes hydrogen or alkyl of 1 to 5 carbon atoms,
$X_m$(m=from 1 to 5) is from one to five identical or different substituents selected from the group consisting of halogen, cyano, trifluoromethyl, nitro, $C_1$–$C_4$-alkyl, $C_1$–$C_4$-alkoxy, substituted or unsubstituted phenyl, substituted or unsubstituted phenoxy, substituted or unsubstituted benzyloxy and hydrogen, and
Y is methyleneoxy, oxymethylene, ethylene, ethenylene, ethynylene, carboxymethylene, carbonylamino, methyleneamino or oxygen,
is allowed to act on the fungi or on the areas, plants, materials or seed threatened by fungal attack.

5. Methyl 2-(bromomethyl)-phenylglyoxylate.

6. A compound as claimed in claim 1, wherein $R^1$ is methyl, $R^2$ is hydrogen, $R^3$ is methyl, $X_m$ is hydrogen and Y is methyleneoxy.

7. A compound as claimed in claim 1, wherein $R^1$ is methyl, $R^2$ is hydrogen, $R^3$ is methyl, $X_m$ is hydrogen and Y is oxymethylene.

8. A compound as claimed in claim 1, wherein $R^1$ is methyl, $R^2$ is hydrogen, $R^3$ is methyl, $X_m$ is 3-methoxy and Y is methyleneoxy.

9. A compound as claimed in claim 1, wherein $R^1$ is methyl, $R^2$ is methyl, $R^3$ is methyl, $X_m$ is 4-methyl and Y is oxymethylene.

## Revendications

1. Hydrazones substituées de la formule I

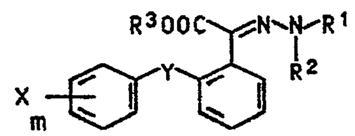

(I)

dans laquelle
$R^1$, $R^2$ et $R^3$ sont identiques ou différents et représentent hydrogène ou alkyle en $C_1$ à $C_5$
X(m=1 à 5) représente des substituants identiques ou différents, halogène, cyano, trifuloorométhyle, nitro, alkyle en $C_1$–$C_4$, alcoxy en $C_1$–$C_4$, phényle éventuellement substitué, phénoxy éventuellement substitué, benzyloxy éventuellement substitué ou hydrogène et
Y représente méthylénoxy, oxyméthylène, éthylène, éthinylène, carboxyméthylène, carbonylamino, méthylamino ou oxygène.

2. Composés de formule I selon la revendication 1 dans laquelle X représente hydrogène, 2-fluoro, 3-fluoro-, 4-fluoro-, 2-chloro-6-fluoro-, 2-chloro-, 3-chloro-, 4-chloro-, 2-bromo-, 3-bromo-, 4-bromo-,

2,4-dichloro-, 2,6-dichloro-, 3,5-dichloro-, 2,4,6-trichloro-, 2-chloro-4-méthyl-, 2-méthyl-4-chloro-, 2-méthyl-, 2–méthyl-4-chloro-, 2-méthyl-, 3 méthyl-, 4-méthyl-, 4-éthyl-, 4-isopropyl-, 4-tert.-butyl-, 2,4-diméthyl-, 2,6-diméthyl-, 2,4,6-triméthyl-, 2-methoxy-4-méthyl-, 4-méthoxy-2-méthyl-, 2-méthoxy-, 3-méthoxy-, 4-méthoxy-, 4-éthoxy-, 4-isopropoxy-, 2-trifluorométhyl-, 4-cyano-, 3-nitro–, 4–nitro–, 4–phényl, 4-benzyloxy-, 4-phénoxy, halogénophénoxy, 4-(2-chloro)-phénoxy-, 4-(2,4-dichloro)-phénoxy-, alkyl en $C_1$–$C_4$ phénoxy, 4-(2-méthyl)-phénoxy-, 3-benzyloxy-, halogèno-benzyloxy-, 3-(2-chloro)-benzyloxy-, 3-(2,4-dichloro)-benzyloxy-, 3-(2-fluoro)-benzyloxy-, 3-(4-bromo)-benzyloxy-, alkyl en $C_1C_4$ benzyloxy-, 3-(2-méthyl)-benzyloxy-, 3-phénoxy-, 3-(2-chloro)-phénoxy-, 3-(2,4-dichloro)-phnoxy-, 3-(2-fluoro)-phénoxy-, 3-(4-bromo)- phénoxy, 3-(2-méthyl)-phénoxy-,

$R^1$ représente hydrogène, méthyle, éthyle, n-propyle, isopropyle, n-butyle, sec.-butyle, isobutyle, tert.-butyle, n-pentyle ou néopentyle

$R^2$ représente hydrogène, méthyle, éthyle, n-propyle, isopropyle, n-butyle, sec.-butyle, isobutyle, tert.-butyle, n-pentyle ou néopentyle,

$R^3$ représente hydrogène, méthyle, éthyle, isopropyle, n-butyle et

Y représente un groupe $-CH_2O-$, $-OCH_2-$, $-CH_2-CH_2-$, $-CH{=}CH-$, –C–C, $-CO_2-CH_2-$, –CO–NH– ou O.

3. Agent fongicide contenant une hydrazone substituée de formule I

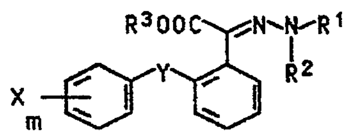

(I)

dans laquelle

$R^1$, $R^2$ et $R^3$ sont identiques ou différents et représentent hydrogène ou alkyle en $C_1$ à $C_5$

X(m=1 à 5) représente des substituants identiques ou différents, halogène, cyano, trifluorométhyle, nitro, alkyle en $C_1$–$C_4$, alcoxy en $C_1$–$C_4$, phényle éventuellement substitué, phénoxy éventuellement substitué, benzyloxy éventuellement substitué ou hydrogène et

Y représente méthylénoxy, oxyméthylène, éthylène, éthinylène, carboxyméthylène, carbonylamino, méthylamino ou oxygène.

4. Procédé de lutte contre les champignons caractérisé par le fait qu'on fait agir sur les champignons ou sur les surfaces, plantes, matériaux ou semences menacées d'une attaque par champignons, une hydrazone substituée de formule I

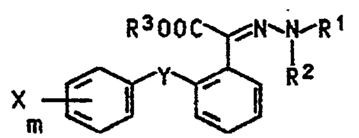

(I)

dans laquelle

$R^1$, $R^2$ et $R^3$ sont identiques ou différents et représentent hydrogène ou alkyle en $C_1$ à $C_5$

X(m=1 à 5) représente des substituants identiques ou différents, halogène, cyano, trifluorométhyle, nitro, alkyle en $C_1$–$C_4$, alcoxy en $C_1$–$C_4$, phényle éventuellement substitué, phénoxy éventuellement substitué, benzyloxy éventuellement substitué ou hydrogène et

Y représente méthylénoxy, oxyméthylène, éthylène, éthinylène, carboxyméthylène, carbonylamino, méthylamino ou oxygène.

5. Ester méthylène d'acide 2-(bromométhyl)-phényl-glyoxylique.

6. Composé de la revendication 1, caractérisé par le fait que $R^1$ représente méthyle, $R^2$, hydrogène, $R^3$, méthyle, Xm, hydrogène et Y, méthylenoxy.

7. Composé selon la revendication 1, caractérisé par le fait que $R^1$ représente méthyle, $R^2$, hydrogène, $R^3$, méthyle, Xm, hydrogène et Y, oxyméthyline;

8. Composé selon la revendication 1, caractérisé par le fait que $R^1$ représente méthyle, $R^2$, hydrogène, $R^3$, méthyle, Xm, 3-méthoxy et Y, méthylénoxy.

9. Composé selon la revendication 1, caractérisé par le fait que $R^1$ représente méthyle, $R^2$, méthyle, $R^3$, méthyle, Xm, 3-méthyle et Y, oxyméthylène.